# EUROPEAN PATENT SPECIFICATION

(11) **EP 2 275 087 B1**
(45) Date of publication and mention of the grant of the patent: **05.09.2012**
(21) Application number: 10154259.5
(22) Date of filing: 22.02.2010
(51) Int. Cl.: A61K 9/16, A61K 9/20, A61K 31/4365

(54) **Prasugrel controlled release formulations**
Formulierungen mit gesteuerter Freisetzung von Prasugrel
Formulations de libération contrôlée de prasugrel

(43) Date of publication of application: 19.01.2011
(73) Proprietor: Helm AG, 20097 Hamburg (DE)
(72) Inventor: Dr. Glänzer, Klaus, 22337, Hamburg (DE)
(74) Representative: Becker Kurig Straus

(56) References cited:
- EP-A1- 2 360 159
- WO-A2-2004/098713
- US-A1- 2007 243 243
- US-A1- 2009 281 136

## Description

### Introduction and background

The present invention relates to a pharmaceutical product for oral administration with controlled release properties regarding pH and release rate. The product comprises prasugrel base in a matrix type pharmaceutical composition which has a dosage form of granules, tablets or, capsules.

The present invention relates also to a pharmaceutical product, which is obtained by
(a) mixing and heating a blend comprising prasugrel particles, one or more waxy compounds having a melting temperature in the range of 40 to 70 °C, and pharmaceutically acceptable excipients selected from the group consisting of diluents, such as lactose or microcrystalline cellulose, and glidants, such as silicon dioxide, at a temperature of from the melting point of said waxy compounds to below the decomposition temperature of prasugrel,
(b) forming a melt granulate or a melt extrudate, cooling to room temperature, and
(c) processing the melt granulate or melt extrudate into prasugrel comprising granular pharmaceutical composition, and optionally
(d) mixing the formed granules with other common additives such as a diluent, a disintegrant, a lubricant, a binder, a colorant, a coating agent, etc, in order to form the desired solid dosage form.

Prasugrel is an adenosine diphosphate (ADP) receptor antagonist, effective in inhibiting platelet activation and aggregation mediated by the platelet P2Y receptor. The drug is used in the treatment or prophylaxis of thrombosis or thrombo-embolism, and related diseases, and generally co-administered with acetylsalicylic acid.

Prasugrel has the chemical name 2-acetoxy-5-(alpha-cyclopropylcarbonbyl-2-fluoro-bencyl)-4,5,6,7-tetrahydrothieno(3,2-c)pyridine and is a prodrug belonging to the group of thienopyridine type compounds, represented by the structural formula I:

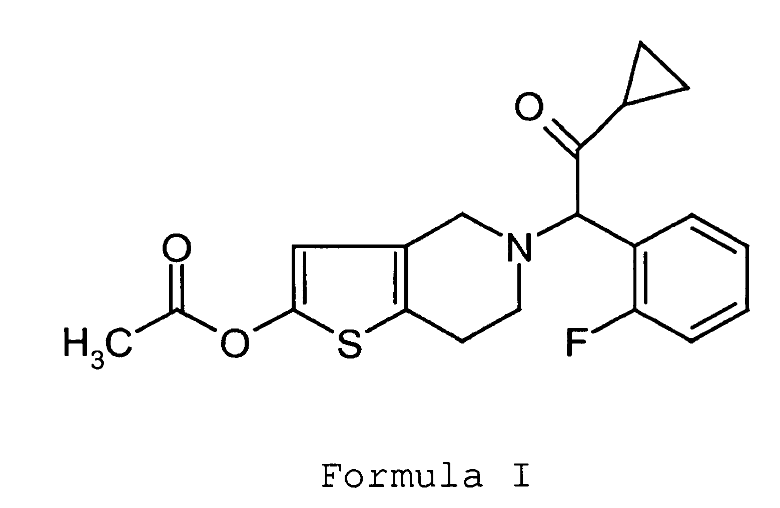

The terminus "prodrug" comprises compounds which itself can be active or inactive, and which can be converted in the human body during their stay into metabolites.

Prasugrel has been described in EP 542411. The compound is a tertiary amine base and forms salts with various acid. Several of these salts have been disclosed (see, e.g., EP 1298132, EP 1728794, EP 2032521, EP 2145890, WO 09/066326, WO 09/098142, or WO 09/129983), of which the hydrochloride is the active ingredient used in the prasugrel containing drug which is marketed under the trade name Efient^{®} by Daiichi-Sankyo and Eli Lilly.

The synthetic route of prasugrel hydrochloride involves three steps according to which production of an intermediate, production of prasugrel free base and the production of prasugrel hydrochloride consecutively takes place.

The pharmacodynamic profile of prasugrel as an anticoagulation agent is believed to result from its rapid absorption and transformation into its active metabolite, which irreversibly blocks ADP-mediated platelet aggregation. The maximum blood concentrations of the metabolite are lower in case that prasugrel is administered with food, or if patients are receiving regular treatment with a proton pump inhibitor, e.g. lansoprazole 30 mg per day.

Clinically significant differences are also seen between results of the administration of prasugrel base or its hydrochloride, particularly in patients undergoing lansoprazole treatment. This effect has been attributed to the strong pH dependence of the solubility of the free base and its salts.

Prasugrel tablets are used in two different ways. In cases where a fast and high level of efficiency regarding inhibition of platelet aggregation is therapeutically needed, a so called "loading dose" of up to 60 mg can be used. The rapid and marked onset of platelet aggregation inhibition is an advantage over other anti-platelet aggregation agents like clopidogrel. In non-acute cases, a so called "maintenance dose" at a level lower than the loading dose is applied. Dosage and frequency of administration are usually selected in a way to optimize the effect on platelet aggregation while minimizing the potential for side effects such as bleeding. The latter side effect depends on age, body weight, medical history, predispositions etc. of the patient. In order to optimize "loading dose" and "maintenance dose" regimens, which is equivalent to minimize potential side effects like bleeding, pharmaceutical formulations are needed which allow for a controlled release of the active ingredient, giving either a very fast release for the loading, or a slow release for the maintenance treatment, with release rates independent of gastric pH.

Due to their chemical structure, prasugrel and its salts are subject to facile hydrolysis or oxidation, leading to stability problems of the active ingredient during manufacturing and storage of the formulated drug (WO 06/135505, EP 1896019). Aqueous solutions of prasugrel are particularly unstable. The stability depends strongly on the pH of the solution. At 25 °C, a maximum of stability has been found for aqueous solutions in the range of pH 4 - 6 [F. Asai et al., Ann. Report Sankyo Res. Lab. 51 (1999)]. For the marketed film coated tablets Efient^{®}, the hydrochloride salt is used because of its improved chemical stability particularly over the free base, but also over other salts. However, Efient^{®} tablets are affected by a progressive salt to base conversion, leading to continuous changes in the physical and chemical properties of the product during storage. These changes are associated with a potential risk due to altered biopharmaceutical behaviour in the way already mentioned above. Obviously, this kind of instability negatively affects the product shelf life.

In attempts to improve stability, prolong shelf life, and ensure clinical efficiency over longer periods of time, the use of other salts or the addition of stabilizing agents (antioxidants and buffer systems) in the formulation have been suggested. Furthermore, the encapsulation or coating of the prasugrel hydrochloride salt particles with cellulose or cellulose derivatives, in particular the use of the water-soluble polymers hydroxypropylmethylcellulose, hydroxypropylcellulose, or polyvinylpyrrolidone, have been reported to improve stability. Finally, the use of a primary packaging of the formulations under inert gas in blister packs impervious to oxygen and moisture, or in tight HDPE containers (see for example EP 1896019, EP 2101767, EP 2100606, EP 2100607, EP 2100608, EP 2100609, EP 2100610, US 2009/0281136) was undertaken to improve the stability of prasugrel comprising tablets.

WO 2004/098713 A2 is concerned with the treatment of cardiovascular diseases and teaches in this regard the use of pharmaceutical compositions comprising prasugrel as the active ingredient in conjunction with an interventional procedure, such as a PCI procedure. The pharmaceutical compositions of WO 2004/098713 A2 may be administered orally or may be coated on a stent, and thus may be formulated with respectively suitable pharmaceutically acceptable excipients (carrier). In line therewith inter alia magnesium stearate, stearic acid and low melting waxes are mentioned.

US 2007/243243 A1 discloses pharmaceutical formulations comprising an antiplatelet agent, e.g. prasugrel, in combination with an acid inhibitor for the treatment of certain gastrointestinal disorders by oral administration. The oral dosage forms may be in the form of powders, granules, tablets or capsules, which may additionally comprise pharmaceutically acceptable excipients. In line therewith, inter alia polyethylene glycol is mentioned together with several other compounds and in connection with several functions of that excipient.

US 2009/0281136 A1 is concerned with the preparation of stable pharmaceutical formulations comprising prasugrel for oral administration. In order to achieve the desired stabilization of the active ingredient US 2009/0281136 A1 teaches several options, such as the use of an antioxidant and/or pharmaceutically acceptable excipients having a low moisture content, coating of tablets comprising prasugrel with gelatine or the preparation of soft gelatine capsules. Alternatively, the pharmaceutical formulation may be packaged in a closed container with a desiccant and an oxygen scavenger.

EP 2 360 159 A1 A1 is a document according to Art. 54(3) EPC and discloses a composition discloses a composition comprising prasugrel and a hydrophilic polymer which can be polyethylene glycol. This composition was made by wet or dry milling of the components.

The approaches introduced to improve the stability profile of prasugrel comprising drugs so far rely on a high level of protective measures, require complicated production or expensive packaging, or a combination of these moves.

If it can be accomplished, the best solution to avoid the therapeutic risk caused by the transformation of the prasugrel hydrochloride and other prasugrel salts into the free base would clearly be the use of prasugrel directly in the form of the free base which is also the active principle. Therefore, there has obviously been a need for a stable pharmaceutical formulation comprising prasugrel free base. Such formulations would have to be easy to manufacture, if possible in a simple and conventional way, and they should have a flexible release profile largely independent on pH. The same kind of approach could then be also applicable for the protective formulation of prasugrel salts.

### Disclosure of the invention

The present invention discloses pharmaceutical formulations comprising prasugrel base characterized by 1.) superior stability, 2.) the possibility to control / program the release of the active ingredient according to therapeutical needs, and 3.) accessibility through a simple and competitive manufacturing process, avoiding in particular the addition of stabilizing agents, or expensive packaging procedures under inert gas. In the manufacturing process according to the present invention, the sensitive particles of prasugrel base are imbedded under mild conditions in different protective low-melting matrices, from which the active ingredient is released at a rate depending on the choice of the matrix.

In this way, the prasugrel formulations of the present invention can be designed and processed in order to obtain pH-independent, fast or slow release drugs. Simple manufacturing processes are e. g. direct compression or thermoplastic granulation (one-pot-melt granulation or melt extrusion carried out under inert gas) prior to the compression into tablets, or prior to the filling into capsules or, for oral granules, into sachets or any other packaging. In these processes, hydrophilic or hydrophobic matrices are used either alone or as mixtures, in order to obtain the requested dissolution profile.

### Detailed description and examples

Therefore, the present invention provides a pharmaceutical product for oral administration with controlled release, comprising a granular pharmaceutical composition which has a dosage form of granules, tablets or capsules, characterized in that said composition comprises
(a) prasugrel particles comprising prasugrel base;
(b) one or more waxy substances or compounds having a melting temperature in the range of 40 to 70 °C, preferably in the range of 50 to 60 °C, selected from the group consisting of pharmaceutical grade polyethylene glycol, glycerol dibehenate, glycerol stearate, and mixtures thereof, at a concentration in the range 2 : 1 to 6 : 1 (w/w) relative to the prasugrel component, or from the group consisting of polyethylene glycol, glycerol dibehenate EP, glycerol distearate EP, and mixtures thereof, imbedding said prasugrel particles in a hydrophilic or hydrophobic matrix governing the release profile ranging from fast to sustained release.

The subclaims contain further embodiments of the invention.

Also the present invention provides a pharmaceutical product, which is obtained by
(a) heating a blend comprising prasugrel particles, one or more waxy compounds having a melting temperature in the range of 40 to 70 °C, and pharmaceutical acceptable excipients selected from the group consisting of diluents, such as, for example, microcrystalline cellulose or lactose, and glidants, such as silicon dioxide, and optionally other functional excipients, e.g. disintegrants (for example Croscarmellose-Na), all of which are common and well known [see: Handbook of Pharmaceutical Excipients], and can appropriately be selected and applied by those skilled in the art, at a temperature of from the melting point of said waxy compounds to below the decomposition temperature of prasugrel,
(b) forming a melt granulate or a melt extrudate, cooling to room temperature, and
(c) processing the melt granulate or melt extrudate into prasugrel comprising granular pharmaceutical composition, and optionally
(d) mixing the formed granules with other common additives such as a diluent, a lubricant, a binder, a glident, a colorant, a coating agent, in order to form the desired solid dosage form. According to an embodiment of the invention the pharmaceutical product has a particle size of the granules obtained by melt granulation or melt extrusion of 200 - 2000 µm.

Although there are no limitations for the usage of pharmaceutically acceptable salts of prasugrel, like its hydrogenchloride, hydrogensulfate, maleate, mesylate, besylate, etc. in the present invention, the investigations leading to the present invention have been foremost concentrated on prasugrel in form of the free base as the active ingredient in the pharmaceutical formulations. This is the most challenging candidate, because it is considered as the most unstable form of prasugrel according to US Patent 6,693,115, and because its solubility is strongly dependent on pH (see F. Asai et al., Ann. Report Sankyo Res. Lab. 51 (1999).

For the preparation of the formulations described in the present invention, particles of prasugrel base (which is a solid of melting point 121-122°C) are embedded in a hydrophilic or a hydrophobic matrix which confers high stability to the formulations. The thermoplastic granulation processes are carried out at temperatures below 75 °C and under a protective inert atmosphere, but all further manufacturing steps no longer require this protection. The formulations based on the direct compression process do not require this kind of protection at all.

According to the present invention one or more waxy compounds having a melting temperature in the range of 40 to 70 °C can be used for imbedding the prasugrel particles in hydrophilic or hydrophobic matrices.

According to a preferred embodiment of the present invention, polyethyleneglycol (Macrogol EP) exhibiting a melting temperature in the range between 40 and 70 °C can be used as a hydrophilic type matrix for prasugrel, while glycerol dibehenate EP and Glycerol distearate EP which exhibit the same range of melting temperatures can be used as hydrophobic type matrices. Through this embedding, both prasugrel base and its salts are finely dispersed in the matrices. For the dispersions, fine particles of less than 80 µm in diameter are preferred in order to ensure complete and proper embedding into these matrices and also fast absorption upon their release. The pH of the formulations is determined by the pH of the matrix and can thus be kept in the range of highest stability (at ca. pH 4 - 6).The waxy matrix components may contain traces of residual peroxides, which potentially could affect the stability of materials sensitive to oxidation, like prasugrel. A respective study has been carried out. Table 3 gives an overview of the content of prasugrel base which was initially present in the respective molten (liquid) matrix at 10 % (w/w) concentration, after storage in open containers at 40 °C for up to 14 days. The samples were analyzed by HPLC, the technique described below under "Analytical Methods". Surprisingly, solutions of prasugrel in Macrogol (polyethylenglycol) with melting points above 40 °C were found to be very stable, which was not the case for a Macrogol matrix being liquid at ambient temperature. Such liquid phases have been suggested as potential liquid vehicles for prasugrel to be filled into soft gelatine capsules (US 2009-281136). Also other predominantly lipohilic type matrices do -surprisingly- not show favourable results regarding the stability of prasugrel (see Table 3).

Along with prasugrel base and the matrix component, the formulations may also contain common pharmaceutical excipients (see Examples). Preferred excipients are those characterised by a low moisture or water content, most preferred of less than 2.0 % (determined by Karl Fischer's method, or "Loss on Drying", as described in Ph. Eur.).

### Examples

Prasugrel base can be produced according to a synthesis disclosed in EP 542411. Its physical and chemical properties have been described by F. Asai et al., Ann. Report Sankyo Res. Lab. 51 (1999). Assay and purity of prasugrel starting material and of the prasugrel comprising products described below was determined by the HPLC method described under *Methods.* On a dry basis, the assay of the prasugrel starting material showed a content of more than 99.9%. The particle size was below 80 µm.

The matrices can be produced by different techniques. The most economic processes are "Single Pot" melt granulation, melt extrusion, and matrix formation by direct compression. Although other techniques, e.g. thermoplastic granulation in a fluid bed granulator, can also be used, the former methods were chosen in the present study for their simplicity and efficiency.

### Formulation by "Single Pot" granulation - Examples 1 - 10

The matrix granulates obtained in Examples 1 - 10 were produced by "Single pot" processing in an inert atmosphere (nitrogen) in a heat jacketed processing plant (Oystar-Hüttlin Processing Plant Unymix). The process cycle includes the following steps: Filling of the components listed in the examples into the container of the processor ; Evacuation and providing inert gas(ca. 1 atm nitrogen); Heating under gentle mixing; Granulation; Cooling; Discharge; Sieving of Granules (e.g., Quadro CoMil, using a suitable screen size). Overall processing times are less than 2 hours per batch.

### Example 1

| Composition | % (w/w) |
|---|---|
| Prasugrel base | 4.0 |
| Lactose EP | 75.5 |
| Macrogol 6000 EP | 20.0 |
| Colloidal silicon dioxide EP | 0.5 |

The melt agglomeration takes place at a product temperature in the range 55 - 60 °C. Within approximately 10 - 15 minutes, homogeneous granules are formed. After cooling and sieving (1.5 mm screen, QuadroComil), a free flowing white to off-white powder is obtained.

### Example 2

| Composition | % (w/w) |
|---|---|
| Prasugrel base | 4.0 |
| Microcrystalline cellulose EP | 75.5 |
| Macrogol 6000 EP | 20.0 |
| Colloidal silicon dioxide EP | 0.5 |

The melt agglomeration takes place at a product temperature in the range 55 - 60 °C. Within approximately 10 - 15 minutes, homogeneous granules are formed. After cooling and sieving (1.5 mm screen, QuadroComil), a free flowing white to off-white powder is obtained.

### Example 3

| Composition | % (w/w) |
|---|---|
| Prasugrel base | 4.0 |
| Lactose EP | 75.5 |
| Macrogol 4000 EP | 20.0 |
| Colloidal silicon dioxide EP | 0.5 |

The melt agglomeration takes place at a product temperature in the range 50 - 55 °C. Within approximately 10 - 15 minutes, homogeneous granules are formed. After cooling and sieving (1.5 mm screen, QuadroComil), a free flowing white to off-white powder is obtained.

### Example 4

| Composition | % (w/w) |
|---|---|
| Prasugrel base | 4.0 |
| Microcrystalline cellulose EP | 75.5 |
| Macrogol 4000 EP | 20.0 |
| Colloidal silicon dioxide EP | 0.5 |

The melt agglomeration takes place at a product temperature in the range 50 - 55 °C. Within approximately 10 - 15 minutes, homogeneous granules are formed. After cooling and sieving (1.5 mm screen, QuadroComil), a free flowing white to off-white powder is obtained.

### Example 5

| Composition | % (w/w) |
|---|---|
| Prasugrel base | 4.0 |
| Microcrystalline cellulose EP | 85.5 |
| Macrogol 6000 EP | 10.0 |
| Colloidal silicon dioxide EP | 0.5 |

The melt agglomeration takes place at a product temperature in the range 55 - 60 °C. Within approximately 10 - 15 minutes, homogeneous granules are formed. After cooling and sieving (1.5 mm screen, QuadroComil), a free flowing white to off-white powder is obtained.

### Example 6

| Composition | % (w/w) |
|---|---|
| Prasugrel base | 4.0 |
| Microcrystalline cellulose EP | 75.5 |
| Glycerol dibehenate EP (Compritol 888^{®}) | 20.0 |
| Colloidal silicon dioxide EP | 0.5 |

The melt agglomeration takes place at a product temperature in the range 55 - 65 °C. Within approximately 10 - 15 minutes, homogeneous granules are formed. After cooling and sieving (2 steps, 2.5mm 1.5 mm screen, QuadroComil), a free flowing white to off-white powder is obtained.

### Example 7

| Composition | % (w/w) |
|---|---|
| | |
| Prasugrel base | 4.0 |
| Lactose EP | 75.5 |
| Glycerol dibehenate EP | 20.0 |
| (Compritol 888^{®}) | |
| Colloidal silicon dioxide EP | 0.5 |

The melt agglomeration takes place at a product temperature in the range 55 - 65 °C. Within approximately 10 - 15 minutes, homogeneous granules are formed. After cooling and sieving(2 steps, 2.5 and 1.5 mm screen, QuadroComil) a free flowing white to off-white powder is obtained.

### Example 8

| Composition | % (w/w) |
|---|---|
| | |
| Prasugrel base | 4.0 |
| Microcrystalline cellulose EP | 75.5 |
| Glycerol distearate EP | 20.0 |
| (Precirol ATO 5^{®}) | |
| Colloidal silicon dioxide EP | 0.5 |

The melt agglomeration takes place at a product temperature in the range 55 - 70 °C. Within approximately 10 - 15 minutes, homogeneous granules are formed. After cooling and sieving(2 steps, 2.5 and 1.5 mm screen, QuadroComil), a free flowing white to off-white powder is obtained.

### Example 9

| Composition | % (w/w) |
|---|---|
| | |
| Prasugrel base | 4.0 |
| Lactose EP | 70.5 |
| Glycerol dibehenate EP | 10.0 |
| (Compritol 888^{®}) | |
| Gelucire 44/14^{®} | 5.0 |
| Colloidal silicon dioxide EP | 0.5 |

The melt agglomeration takes place at a product temperature in the range 50 - 60 °C. Within approximately 10 - 15 minutes, homogeneous granules are formed. After cooling and sieving (2 steps, 2.5 and 1.5 mm screen, QuadroComil), a free flowing white to off-white powder is obtained.

### Example 10

| Composition | % (w/w) |
|---|---|
| | |
| Prasugrel base | 4.0 |
| Lactose EP | 70.5 |
| Glycerol dibehenate EP | 5.0 |
| (Compritol 888^{®}) | |
| Glycerol distearate EP | 5.0 |
| (Precirol ATO 5^{®}) | |
| Colloidal silicon dioxide EP | 0.5 |

The melt agglomeration takes place at a product temperature in the range 55 - 65 °C. Within approximately 10 - 15 minutes, homogeneous granules are formed. After cooling and sieving (2 steps, 2.5 and 1.5 mm screen, QuadroComil), a free flowing white to off-white powder is obtained.

### Extrusion granulates - Examples 11 - 14

Matrix granulates of examples 11 - 14 were produced by conventional melt extrusion under inert gas (nitrogen) atmosphere (in a Leistritz Hot Melt Extrusion Plant). The process cycle includes the following steps: Blending; Inertisation; Feeding; Hot Melt Extrusion; Cooling; Comminution and Sieving. Depending on equipment, 2 - 3 kg of granules are obtained per hour.

### Example 11

| Composition | % (w/w) |
|---|---|
| | |
| Prasugrel base | 10.0 |
| Microcrystalline cellulose EP | 64.5 |
| Macrogol 4000 EP | 25.0 |
| Colloidal silicon dioxide EP | 0.5 |

The melt extrusion takes place at a product temperature in the range 50 - 70 °C. After cooling and sieving (2 steps, 2.5 and 1.5 mm screen, QuadroComil), a free flowing white to off-white powder is obtained.

### Example 12

| Composition | % (w/w) |
|---|---|
| | |
| Prasugrel base | 10.0 |
| Lactose EP | 59.5 |
| Macrogol 6000 EP | 30.0 |
| Colloidal silicon dioxide EP | 0.5 |

The melt extrusion takes place at a product temperature in the range 55 - 70 °C. After cooling and sieving (2 steps, 2.5 and 1.5 mm screen, QuadroComil), a free flowing white to off-white powder is obtained.

### Example 13

| Composition | % (w/w) |
|---|---|
| | |
| Prasugrel base | 10.0 |
| Lactose EP | 69.5 |
| Glycerol dibehenate EP | 20.0 |
| (Compritol 888^{®}) | |
| Colloidal silicon dioxide EP | 0.5 |

The melt extrusion takes place at a product temperature in the range 65 - 75 °C. After cooling and sieving (2 steps, 2.5 and 1.5 mm screen, QuadroComil), a free flowing white to off-white powder is obtained.

### Example 14

| Composition | % (w/w) |
|---|---|
| | |
| Prasugrel base | 10.0 |
| Microcrystalline cellulose EP | 49.5 |
| Glycerol dibehenate EP | 40.0 |
| (Compritol 888^{®}) | |
| Colloidal silicon dioxide EP | 0.5 |

The melt extrusion takes place at a product temperature in the range 65 - 75 °C. After cooling and sieving (2.5 and 1 mm screen, QuadroComil), a free flowing white to off-white powder is obtained.

### Matrix tablets by direct compression - Examples 15 -17

Matrix tablets of examples 15 - 17 were produced as follows: The active ingredient and the matrix building components are premixed in a bin type blender (e.g., 10 min, 5-10 rpm) and sieved (Frewitt 1.0 mm screen). The premix is added to the excipients, and homogeneously mixed (typical mixing times 10 - 15 minutes). The blend is compressed on a conventional rotary press (Fette P 1200) to tablets (round, biconvex, 9 mm diameter, target weight 250 mg). Typical speed was in the range 30 000 - 50 000 tablets per hour.

### Example 15

| Composition | mg/ tablet |
|---|---|
| | |
| Prasugrel base | 10.0 |
| Lactose EP | 188.75 |
| Glycerol dibehenate EP | 50.0 |
| (Compritol 888^{®}) | |
| Colloidal silicon dioxide EP | 1.25 |

White to off-white tablets; hardness: 55 - 69 N; friability < 0.1 %

### Example 16

| Composition | mg/tablet |
|---|---|
| | |
| Prasugrel base | 10.0 |
| Lactose EP | 173.75 |
| Macrogol 6000 EP | 50.0 |
| Crospovidone Type A EP | 15.0 |
| Colloidal silicon dioxide EP | 1.25 |

White to off-white tablets; Hardness: 48 - 63 N; Friability < 0.1 %

### Example 17

| Composition | mg/tablet |
|---|---|
| | |
| Prasugrel base | 10.0 |
| Lactose EP | 208.75 |
| Glycerol dibehenate EP | 15.0 |
| (Compritol 888^{®}) | |
| Macrogol 6000 EP | 15.0 |
| Colloidal silicon dioxide EP | 1.25 |

White to off-white tablets; hardness: 56 - 69 N; friability < 0.1 %

### Tablets by compression of matrix granulates - Examples 18 - 23

The tablets of examples 18 - 23 were produced as follows: The prasugrel-containing matrix granulates of the respective examples, and the other excipients are mixed in a bin type blender (typical mixing times 10 - 15 min). In Example 22, the Prasugrel base was added after being premixed with Glyceroldibehenate EP. In case that a lubricant like Magnesium stearate EP was used, this was added to the blend at the final step, and homogeneously mixed again (typical mixing times 5 minutes). The final blend was compressed on a conventional rotary press (Fette P 1200) to tablets (round, biconvex, 9 or 10 mm diameter, target weight of 240 - 280 mg or 555 mg, respectively, depending on the prasugrel content of the granulate). Typical speed was in the range 30 000 - 50 000 tablets per hour.

### Example 18

| Composition | mg/ tablet |
|---|---|
| | |
| Granulates of example 7 | 244 |
| (equivalent to 10 mg Prasugrel base) | |
| Magnesium stearate EP | 1.0 |

White to off-white tablets, 245 mg; hardness: 45 - 55 N; friability 0.10 %

### Example 19

| Composition | mg/ tablet |
|---|---|
| | |
| Granulates of example 7 | 244 |
| (equivalent to 10 mg Prasugrel base) | |
| Acetylsalicylic acid EP | 200 |
| Microcrystalline cellulose EP | 50 |
| Magnesium stearate EP | 1.0 |

White to off-white tablets, 555 mg; hardness: 75 - 85 N; friability 0.16 %

### Example 20

| Composition | mg/ tablet |
|---|---|
| | |
| Granulates of example 8 | 261 |
| (equivalent to 10 mg Prasugrel base) | |
| Magnesium stearate EP | 1.0 |

White to off-white tablets, 262 mg; hardness: 45 - 55 N; friability 0.10 %

### Example 21

| Composition | mg/ tablet |
|---|---|
| | |
| Granulates of Example 1 | 259 |
| (equivalent to 10 mg Prasugrel base) | |
| Crospovidone Type A | 15 |
| Magnesium stearate EP | 1.0 |

White to off-white tablets, 275 mg; hardness: 45 - 50 N; friability 0.14 %

### Example 22

| Composition | mg/tablet |
|---|---|
| | |
| Granulates of Example 2 | 255 |
| (equivalent to 10 mg Prasugrel base) | |
| Crospovidone Type A | 15 |
| Magnesium stearate EP | 1.0 |

White to off-white tablets, 271 mg; hardness: 45 - 55 N; friability < 0.10 %

### Example 23

| Composition | mg/ tablet |
|---|---|
| | |
| Granulates of Example 1 | 130.0 |
| (equivalent to 5 mg Prasugrel base) | |
| Prasugrel base | 5.0 |
| Lactose EP | 104.0 |
| Glycerol dibehenate EP | 10.0 |
| (Compritol 888^{®}) | |
| Colloidal silicon dioxide EP | 0.5 |
| Magnesium stearate EP | 0.5 |

White to off-white tablets, 250 mg; hardness: 45 - 55 N; friability <0.10 %

### Matrix granulates in capsules - Example 24

### Example 24

259 mg (or 130 mg) granulates of Example 1, equivalent to 10 mg (or 5 mg) Prasugrel base, are filled directly into capsules of size 1 (or size 2, respectively).

### Matrix granulates as oral granules - Example 25

### Example 25

| Composition | mg/ unit dose |
|---|---|
| | |
| Granulates of Example 7 | 244.0 |
| (equivalent to 10 mg Prasugrel base) | |
| Sorbitol EP | 2248.5 |
| Colloidal silicon dioxide EP | 7.5 |

The components of Example 25 are mixed (10 - 15 min. in a bin type blender, 30 - 50 rpm), and filled as single dose into sachets.

### Analytical Methods

### 1. HPLC method for prasugrel and related substances (impurities)

HPLC System Agilent 1100; C18 column, 20 °C; UV detection 218 nm; injection volume 10 µl; flow rate 1.2 ml/min, time per run 80 min; Mobile phase: phosphate buffer pH 6.0 / acetonitrile, A = 80/20, B = 20/80); sample preparation: dissolve in acetonitrile; sample solutions are stable for at least 24 h.

### 2.Dissolution Test method

The dissolution was carried out according to Ph. Eur. 2.9.3, paddle, 100 rpm.
Dissolution media (900 ml, 37 °C):
a) pH 1.2 - 0.1 n HCl-solution
b) pH 4.0 - phosphate buffer + 0.5% sodium laurylsulfate solution
c) pH 6.5 - phosphate buffer + 1.0% sodium laurylsulfate solution

### Dissolution Data

Prasugrel base is very unstable in strong acidic (pH < 2) or alkaline (pH > 7) solutions. In case of dissolution medium a) and a temperature of 37 °C (i.e., the temperature to be applied in the dissolution test), more than 5 % decomposition is seen after 15 min test time. In case of dissolution medium b) and a temperature of 37 °C, more than 5 % of prasugrel are decomposed after 1 hour test time. In case of dissolution medium c) and T = 37 °C,a decomposition of 2 - 3 % after 6 hours test time is observed. Unfortunately, the range of better stability is also a range of minimum solubility of prasugrel in aqueous solutions. In order to achieve sufficient solubility, a solubility enhancer (sodium laurylsulfate) has been added to the test medium. Since the solubility of prasugrel is high enough at lower pH values, no solubility enhancement is needed. The conditions and test times to determine the dissolution data of the following tables were selected in a way that an influence of the decomposition of prasugrel can be neglected.

**Table 1**

| pH Dependence of prasugrel release in case of "fast release" formulations. The dissolution test data indicate the quantity of prasugrel released after 5 min: | | | |
|---|---|---|---|
| Dissolution Medium | Efient 10 mg Tablets batch A 594158 | Tablets Example 21 | Tablets Example 16 |
| pH 1.2 (a) | 98 % | 24 % | 45 % |
| pH 4.0 (b) | 58 % | 22 % | 44 % |
| pH 6.5 (c) | 26 % | 20 % | 46 % |

**Table 2**

| Comparison of release profiles (dissolution test medium pH 6.5 (c)). The data indicate the released quantity (%) of prasugrel at the respective sampling time. | | | | | | |
|---|---|---|---|---|---|---|
| Sampling time (h) | Efient Tabl.10 mg batch A594158 | Tablets Example 16 | Tablets Example 21 | Tablets Example 17 | Tablets Example 15 | Tablets Example 18 |
| ½ | 88 | 94 | 71 | 49 | 6 | 1 |
| 1 | 92 | 93 | 81 | 61 | 11 | 2 |
| 2 | | | | 68 | 29 | 3 |
| 3 | | | | 81 | 40 | 5 |
| 4 | | | | 90 | 49 | 6 |
| 24 | | | | | (91)* | (35)* |

| | | | | | | |
|---|---|---|---|---|---|---|
| * 24 hour data not corrected for prasugrel decomposition | | | | | | |

### Stability Data

**Table 3**

| Stability overview of prasugrel base dissolved (embedded) at a concentration of 1 g per 10 g matrix, in hydrophilic and hydrophobic matrices, respectively. The data indicate the amount of initial prasugrel left after storage at 40°C over 7 and 14 days. | | | |
|---|---|---|---|
| Matrix/Solvent | Storage time: | 7 days | 14 days |
| Lutrol F 68^{®} | | 95.85 | |
| (Poloxamer 188) | | | |
| | | | |
| Solutol HS 15^{®} | | 16.3 | |
| (Macrogol-15-hydroxystearate) | | | |
| Macrogol 400 EP | | 87.3 | |
| (Freezing point 4 - 8 °C) | | | |
| | | | |
| Macrogol 6000 EP | | 99.6 | 99.6 |
| | | | |
| Compritol 888^{®} | | 100.2 | 100.2 |
| (Glycerol dibehenate EP) | | | |
| | | | |
| Precirol ATO 5^{®} | | 99.8 | 99.9 |
| (Glycerol distearate EP) | | | |
| | | | |
| Macrogol 6000 EP (90 %) | | 97.1 | 96.9 |
| + Gelucire 44/14^{®} (10%) | | | |
| (Lauroyl Macrogol-32 glycerides EP) | | | |

**Table 4**

| Stability overview of prasugrel granulates. The data indicate the content of prasugrel (mg prasugrel / g) and the amount of total impurities (% of peak surface relative to prasugrel peak) as determined by HPLC: initial results, and results after open storage at ambient temperature over 1 and 3 months, respectively. | | | |
|---|---|---|---|
| Sample | Assay (mg /g); Sum of impurities (%) | | |
| | Initial | 1 month | 3 months |
| | | | |
| Granulate | 37.2 mg/g | 37.4 mg/g | 36.8 mg/g (= 98.9 % of initial) |
| Example 1 | 0.74 % | 0.70 % | 0.81 % |
| | | | |
| Granulate | 38.6 mg/g | 37.5 mg/g | 37.5 mg/g (= 97.5 % of initial) |
| Example 2 | 0.83 % | 0.87 % | 1.05 % |
| Granulate | 41.5 mg/g | 40.7 mg/g | 41.5 mg/g (=100.0 % of initial) |
| Example 7 | 0.48 % | 0.57 % | 0.57 % |

**Table 5**

| Stability overview of prasugrel tablets. The data indicate the content of prasugrel (mg prasugrel / tablet) and the amount of total impurities (% of peak surface relative to prasugrel peak) as determined by HPLC: initial results, and results after open storage at ambient temperature over 3 months, respectively. | | |
|---|---|---|
| Sample | Assay (mg /tablet); Sum of impurities (%) | |
| | Initial | 3 months |
| | | |
| Tablets | 9.59 mg/tablet | 9.59 mg/tablet (= 100.0 % of initial) |
| Example 15 | 0.66 % | 0.77 % |
| | | |
| Tablets | 9.91 mg/tablet | 9.98 mg/tablet (= 100.7 % of initial) |
| Example 16 | 0.61 % | 0.71 % |
| | | |
| Tablets | 9.14 mg/tablet | 9.28 mg/tablet (= 101.5 % of initial) |
| Example 18 | 0.56 % | 0.58 % |
| | | |
| Tablets | 10.14 mg/tablet | 10.42 mg/tablet (= 102.8 % of initial) |
| Example 21 | 1.04% | 1.08 % |

### Key Observations of the Present Invention

The present invention discloses a simple and efficient way to formulate and produce prasugrel base comprising oral pharmaceutical products allowing for the design of specific release profiles, without dependence on the pH, in order to fulfil certain therapeutic requirements regarding an individual loading dose or maintenance dose, respectively, and to minimize unwanted side effects.

Surprisingly, this can be achieved by imbedding the prasugrel particles in hydrophilic or hydrophobic matrices, as described in the examples. Depending on mixing ratio and choice of the technology, a tailor-made release profile can be achieved. The same procedure can be used for formulations with therapeutically acceptable salts of prasugrel.

The following aspects are of special relevance:
a) At a given level/mixing ratio of matrix material, the release rate from tablets obtained by the direct compression technology is faster than from those obtained via melt granulation or melt extrusion technology.
b) Use of a pure hydrophilic matrix, or a high mixing ratio of hydrophilic matrix material leads to the fastest release rates, whereas pure hydrophobic matrices, or a high mixing ratio of hydrophobic matrix material leads to most sustained release.

The design of fast and pH independent release is obvious from Examples 16 and 21 (see Table 1): Whereas Efient^{®} tablets are characterized by a strong dependence on pH in the dissolution media (approximately 4 times faster release rate at strongly acidic conditions of pH 1.2 compared with the release at pH 6.5), the tablets of Example 16 and Example 21 are almost constant over the whole pH range, with an average release of 22 % or 45 % after 5 minutes, respectively. This type of prasugrel formulation is particularly suitable for loading doses.

The effect of the proper design of formulations for immediate, or more or less sustained release, is obvious from the examples given in Table 2: The fast release tablets of Example 16 are showing more than 90 % release after 1/2 hour. The tablets of the Examples 21, 17, 15, and 18 are characterized by decreasing dissolution rates, extending from medium to slow, and very slow dissolution rates. Prasugrel formulations obtained in these examples are suited to avoid peak levels of the active metabolite, i.e. for maintenance dosing.

The good stability of prasugrel granulates and prasugrel tablets is shown in Tables 4 and 5, respectively. Surprisingly, no protective measures like adding antioxidants or buffers, providing a low humidity environment, or packaging into airtight blisters or containers under nitrogen and with adsorbents, are needed. The stability of prasugrel base is not so much dependent on the nature of the matrices of the present invention, however, the quantity of matrix material must be sufficient to cover all the prasugrel base particles. In order to ensure complete embedding, the matrix material must be at least 2 times the quantity (w/w) of prasugrel.

## Claims

1. A pharmaceutical product for oral administration with controlled release, comprising a granular pharmaceutical composition which has a dosage form of granules, tablets or capsules, wherein the said composition comprises
(a) prasugrel particles comprising prasugrel base;
(b) one or more waxy substances or compounds having a melting temperature in the range of 40 to 70 °C, selected from the group consisting of pharmaceutical grade polyethylene glycol, glycerol dibehenate, glycerol stearate, and mixtures thereof, at a concentration in the range 2 : 1 to 6 : 1 (w/w) relative to the prasugrel component, or from the group consisting of polyethylene glycol, glycerol dibehenate EP, glycerol distearate EP, and mixtures thereof, imbedding said prasugrel particles in a hydrophilic or hydrophobic matrix governing the release profile ranging from fast to sustained release.

2. The pharmaceutical product according to claim 1, wherein the matrix forming waxy compounds have a melting temperature in the range of 50 to 60 °C.

3. The pharmaceutical product according to claim 1 or 2, wherein the matrix forming waxy compounds are selected from the group consisting of pharmaceutical grade polyethylene glycol, glycerol dibehenate, glycerol stearate, and mixtures thereof, at a concentration in the range 3 : 1 to 5 : 1 (w/w) relative to the prasugrel component.

4. The pharmaceutical product according to any one of the previous claims 1 to 3, wherein the prasugrel particles have a particle size of less than 80 µm, preferably of less than 40 µm, most preferably of less than 20 µm.

5. The pharmaceutical product according to any one of the previous claims 1 to 4, to which one or more active ingredients, preferably acetylsalicylic acid, and various excipients are added to the external or matrix phase.

6. The pharmaceutical product according to any one of the previous claims 1 to 5, which is obtained by
(a) heating a blend comprising the prasugrel particles, the one or more waxy compounds, and pharmaceutical acceptable excipients selected from the group consisting of diluents, such as microcrystalline cellulose or lactose, and glidants, such as silicon dioxide, and optionally other functional components, at a temperature of from the melting point of said waxy compounds to below the decomposition temperature of prasugrel,
(b) forming a melt granulate or a melt extrudate, cooling to room temperature, and
(c) processing the melt granulate or melt extrudate into said prasugrel comprising granular pharmaceutical composition, and optionally
(d) mixing the formed granules with other common additives such as a diluent, a lubricant, a binder, a glidant, a colorant, a coating agent,
in order to form the desired solid dosage form.

7. The pharmaceutical product according to claim 6, wherein the particle size of the granules obtained by melt granulation or melt extrusion is 200 - 2000 µm.

8. A process for the production of the pharmaceutical product according to any one of the previous claims 1 to 7, comprising the steps of
(a) heating a blend comprising the prasugrel particles, the one or more waxy compounds, and pharmaceutical acceptable excipients selected from the group consisting of diluents, such as microcrystalline cellulose or lactose, and glidants, such as silicon dioxide, and optionally other functional components, at a temperature of from the melting point of said waxy compounds to below the decomposition temperature of prasugrel,
(b) forming a melt granulate or a melt extrudate, cooling to room temperature, and
(c) processing the melt granulate or melt extrudate into said prasugrel comprising granular pharmaceutical composition, and optionally
(d) mixing the formed granules with other common additives such as a diluent, a lubricant, a binder, a glidant, a colorant, a coating agent, in order to form the desired solid dosage form.

## Patentansprüche

1. Pharmazeutisches Erzeugnis mit gesteuerter Freisetzung zur oralen Verabreichung, umfassend eine granuläre pharmazeutische Zusammensetzung, welches in Form von Granulaten, Tabletten oder Kapseln dosiert ist, wobei die Zusammensetzung umfasst
(a) Prasugrel-Teilchen, welche Prasugrel-Base umfassen;
(b) eine oder mehrere wachsartige Substanzen oder Verbindungen mit einer Schmelztemperatur im Bereich von 40 bis 70 °C, welche ausgewählt ist/sind aus der Gruppe, bestehend aus pharmazeutisch reinem Polyethylenglykol, Glycerindibehenat, Glycerinstearat und Mischungen davon, in einem Konzentrationsbereich von 2 : 1 bis 6 : 1 (Gew./Gew.), bezogen auf die Prasugrel-Komponente, oder aus der Gruppe, bestehend aus Polyethylenglykol, Glycerindibehenat EP, Glycerindistearat EP und Mischungen davon, welche die Prasugrel-Teilchen in eine hydrophile oder hydrophobe Matrix einbetten, die das Freisetzungsprofil im Bereich von schnell bis verzögerte Freigabe bestimmt.

2. Pharmazeutisches Erzeugnis nach Anspruch 1, wobei die Matrix-bildenden wachsartigen Verbindungen eine Schmelztemperatur im Bereich von 50 bis 60 °C aufweisen.

3. Pharmazeutisches Erzeugnis nach Anspruch 1 oder 2, wobei die Matrix-bildenden wachsartigen Verbindungen ausgewählt sind aus der Gruppe, bestehend aus pharmazeutisch reinem Polyethylenglykol, Glycerindibehenat, Glycerinstearat und Mischungen davon, in einem Konzentrationsbereich von 3 : 1 bis 5 : 1 (Gew./Gew.), bezogen auf die Prasugrel-Komponente.

4. Pharmazeutisches Erzeugnis nach einem der vorhergehenden Ansprüche 1 bis 3, wobei die Prasugrel-Teilchen eine Teilchengröße von weniger als 80 µm, vorzugsweise von weniger als 40 µm, am bevorzugtesten von weniger als 20 µm aufweisen.

5. Pharmazeutisches Erzeugnis nach einem der vorhergehenden Ansprüche 1 bis 4, zu dem ein oder mehrere Wirkstoffe, vorzugsweise Acetylsalicylsäure, und verschiedene Exzipienten zu der äußeren Phase oder der Matrixphase zugesetzt sind.

6. Pharmazeutisches Erzeugnis nach einem der vorhergehenden Ansprüche 1 bis 5, welches erhalten wird durch
(a) Erwärmen einer Mischung, umfassend die Prasugrel-Teilchen, die eine oder die mehreren wachsartigen Verbindungen und pharmazeutisch annehmbare Exzipienten, welche ausgewählt sind aus der Gruppe, bestehend aus Diluenten, wie mikrokristalline Cellulose oder Lactose, und Gleitmitteln, wie Siliciumdioxid, und gegebenenfalls andere funktionelle Komponenten, bei einer Temperatur von vom Schmelzpunkt der wachsartigen Verbindungen bis unterhalb der Zersetzungstemperatur von Prasugrel,
(b) Bilden von Schmelzgranulat oder Schmelzextrudat, Abkühlen auf Raumtemperatur, und
(c) Verarbeiten des Schmelzgranulats oder Schmelzextrudats in die Prasugrel umfassende granuläre pharmazeutische Zusammensetzung, und gegebenenfalls
(d) Mischen der gebildeten Granulate mit anderen üblichen Zusatzstoffen, wie einem Diluenten, einem Lubrikans, einem Bindemittel, einem Gleitmittel, einem Farbstoff, einem Beschichtungsmittel, um die gewünschte feste Dosierungsform zu bilden.

7. Pharmazeutisches Erzeugnis nach Anspruch 6, wobei die Teilchengröße der durch Schmelzgranulation oder Schmelzextrusion erhaltenen Granulate 200 bis 2000 µm beträgt.

8. Verfahren zur Herstellung des pharmazeutischen Erzeugnisses nach einem der vorhergehenden Ansprüche 1 bis 7, umfassend die Schritte von
(a) Erwärmen einer Mischung, umfassend die Prasugrel-Teilchen, die eine oder die mehreren wachsartigen Verbindungen und pharmazeutisch annehmbare Exzipienten, welche ausgewählt sind aus der Gruppe, bestehend aus Diluenten, wie mikrokristalline Cellulose oder Lactose, und Gleitmitteln, wie Siliciumdioxid, und gegebenenfalls andere funktionelle Komponenten, bei einer Temperatur von vom Schmelzpunkt der wachsartigen Verbindungen bis unterhalb der Zersetzungstemperatur von Prasugrel,
(b) Bilden von Schmelzgranulat oder Schmelzextrudat, Abkühlen auf Raumtemperatur, und
(c) Verarbeiten des Schmelzgranulats oder Schmelzextrudats in die Prasugrel umfassende granuläre pharmazeutische Zusammensetzung, und gegebenenfalls
(d) Mischen der gebildeten Granulate mit anderen üblichen Zusatzstoffen, wie einem Diluenten, einem Lubrikans, einem Bindemittel, einem Gleitmittel, einem Farbstoff, einem Beschichtungsmittel, um die gewünschte feste Dosierungsform zu bilden.

## Revendications

1. Produit pharmaceutique destiné à une administration orale à libération contrôlée, qui comprend une composition pharmaceutique granulaire sous forme posologique de granules, de comprimés ou de gélules, dans lequel ladite composition comprend :
(a) des particules de prasugrel comprenant une base de prasugrel ;
(b) au moins une substance cireuse ou au moins un composé cireux ayant une température de fusion dans la plage de 40 à 70°C, choisis dans le groupe constitué du polyéthylène glycol de qualité pharmaceutique, du dibéhénate de glycérol, du stéarate de glycérol, et de leurs mélanges, à une concentration dans la plage de 2/1 à 6/1 (p/p) relativement au composant prasugrel, ou dans le groupe constitué du polyéthylène glycol, du dibéhénate de glycérol EP, du distéarate de glycérol EP, et de leurs mélanges, l'intégration desdites particules de prasugrel dans une matrice hydrophile ou hydrophobe régissant le profil de libération dans la plage d'une libération rapide à une libération prolongée.

2. Produit pharmaceutique la revendication 1, dans lequel les composés cireux formant la matrice ont une température de fusion dans la plage de 50 à 60°C.

3. Produit pharmaceutique selon la revendication 1 ou 2, dans lequel les composés cireux formant la matrice sont choisis dans le groupe constitué du polyéthylène glycol de qualité pharmaceutique, du dibéhénate de glycérol, du stéarate de glycérol, et de leurs mélanges, à une concentration dans la plage de 3/1 à 5/1 (p/p) relativement au composant prasugrel.

4. Produit pharmaceutique selon l'une quelconque des revendications 1 à 3, dans lequel les particules de prasugrel ont une taille de particules inférieure à 80 µm, de préférence inférieure à 40 µm, idéalement inférieure à 20 µm.

5. Produit pharmaceutique selon l'une quelconque des revendications 1 à 4, à la phase externe ou matricielle duquel au moins un principe actif, de préférence l'acide acétylsalicylique, et différents excipients sont ajoutés.

6. Produit pharmaceutique selon l'une quelconque des revendications 1 à 5, qui est obtenu par les étapes consistant à :
(a) chauffer un mélange comprenant les particules de prasugrel, le ou les composés cireux, et les excipients pharmaceutiquement acceptables choisis dans le groupe constitué de diluants, tels que la cellulose microcristalline ou le lactose, et d'agents de glissement, tel que le dioxyde de silicium, et facultativement d'autres composants fonctionnels, à une température comprise entre le point de fusion desdits composés cireux et une température inférieure à la température de décomposition du prasugrel,
(b) former un granulat fondu ou un extrudat fondu, refroidir à température ambiante, et
(c) transformer le granulat fondu ou l'extrudat fondu en ladite composition pharmaceutique granulaire comprenant du prasugrel, et facultativement
(d) mélanger les granules formés avec d'autres additifs courants tels qu'un diluant, un lubrifiant, un liant, un agent de glissement, un colorant, un agent d'enrobage, de manière à obtenir la forme posologique solide souhaitée.

7. Produit pharmaceutique selon la revendication 6, dans lequel la taille de particules des granules obtenus par granulation en fusion ou extrusion en fusion est de 200 à 2000 µm.

8. Procédé de production du produit pharmaceutique selon l'une quelconque des revendications 1 à 7, comprenant les étapes consistant à :
(a) chauffer un mélange comprenant les particules de prasugrel, le ou les composés cireux, et les excipients pharmaceutiquement acceptables choisis dans le groupe constitué de diluants, tels que la cellulose microcristalline ou le lactose, et d'agents de glissement, tel que le dioxyde de silicium, et facultativement d'autres composants fonctionnels, à une température comprise entre le point de fusion desdits composés cireux et une température inférieure à la température de décomposition du prasugrel,
(b) former un granulat fondu ou un extrudat fondu, refroidir à température ambiante, et
(c) transformer le granulat fondu ou l'extrudat fondu en ladite composition pharmaceutique granulaire comprenant du prasugrel, et facultativement
(d) mélanger les granules formés avec d'autres additifs courants tels qu'un diluant, un lubrifiant, un liant, un agent de glissement, un colorant, un agent d'enrobage, de manière à obtenir la forme posologique solide souhaitée.
